# EUROPEAN PATENT APPLICATION

(11) **EP 3 441 068 A1**
(43) Date of publication of application: **13.02.2019**
(21) Application number: 17796255.2
(22) Date of filing: 12.05.2017
(51) Int. Cl.: A61K 31/496, A61K 47/12, A61K 47/14, A61K 47/20, A61P 25/18

(54) **TRANSDERMAL PREPARATION**

(30) Priority: 12.05.2016 JP 2016096178; 19.12.2016 JP 2016245702
(71) Applicant: FUJIFILM Corporation, Tokyo 106-8620 (JP)
(72) Inventor: YONEYAMA, Satoshi, Ashigarakami-gun Kanagawa 258-8577 (JP); ENDO, Taisuke, Ashigarakami-gun Kanagawa 258-8577 (JP)
(74) Representative: Klunker IP Patentanwälte PartG mbB
(86) International application number: PCT/JP2017/018086
(87) International publication number: WO 2017/195897

(57) **Abstract**

Provided is a transdermal preparation including: a phenylpiperazine derivative; an organic acid; an ester-based solvent; and an organic sulfoxide.

## Description

### BACKGROUND OF THE INVENTION

### 1. Field of the Invention

The present disclosure relates to a transdermal preparation.

### 2. Description of the Related Art

A phenylpiperazine derivative has been widely used as an antipsychotic drug. Specifically, a phenylpiperazine derivative such as aripiprazole or brexpiprazole has an effect on improvement of schizophrenia, mania or depression in bipolar disorder, or a depression state.

As an example of the phenylpiperazine derivative, it has been suggested that the aripiprazole is used as a drug for treating schizophrenia (for example, see JP2608788B).

JP2608788B describes a phenylpiperazine derivative as an active component. However, in regard to the administration method, the research has been made only on oral administration, injection, and a suppository.

In some cases, it is difficult to periodically administer a drug orally to a person who develops the symptoms of schizophrenia, mania or depression in bipolar disorder, or a depression state. Therefore, in recent years, a dosage form that enables a phenylpiperazine derivative to be transdermally absorbed has been examined in place of oral administration. A transdermal preparation has an advantage that a therapist or a caregiver can easily administer the transdermal preparation to a person developing the symptoms of schizophrenia and confirmation on whether the transdermal preparation exists on the skin can be easily made.

However, a phenylpiperazine derivative has a relatively large molecular weight and is unlikely to be formed into a transdermal preparation. Accordingly, various attempts for applying the phenylpiperazine derivative as a transdermal preparation have been made.

For example, an external agent composition with excellent transdermal absorbability which contains an organic acid such as an aromatic sulfonic acid, an aliphatic sulfonic acid, an aromatic carboxylic acid, or an aliphatic carboxylic acid in an amount of 0.5 molar times to 5 molar times one mole of aripiprazole and also contains aripiprazole as an organic acid salt in the preparation system has been suggested (for example, see WO2010/146872A).

Further, as a preparation that enables an active component to be transdermally absorbed at a high concentration, a transdermal preparation which contains one or more selected from an organic sulfoxide, a fatty acid ester, or a fatty acid with respect to an active component has been suggested (for example, see WO2007/016766A).

### SUMMARY OF THE INVENTION

The external agent composition described in WO2010/146872A enables aripiprazole to be transdermally absorbed through the keratin. However, after the aripiprazole penetrates into the keratin, a coexisting solvent is rapidly diffused into the dermis or the epidermis. As the result, the aripiprazole with a therapeutically effective concentration is unlikely to be continuously supplied for a long certain period of time in some cases.

Further, as the compounds which are considered to be effective for transdermal absorption, WO2007/016766A describes various compounds as an organic sulfoxide, a fatty acid ester, and a fatty acid. However, a phenylpiperazine derivative serving as an active component after transdermal absorption has not been paid particular attention, and the effect of transdermally absorbing a phenylpiperazine derivative and the persistence of the effect have not been examined at all.

An object of an embodiment of the present invention is to provide a transdermal preparation which enables a phenylpiperazine derivative serving as an active component to be transdermally absorbed at a high permeation rate and is capable of maintaining the supply concentration and the supply time of the active component into a living body.

The means for solving the above-described problems includes the following embodiment.
[1] A transdermal preparation comprising: a phenylpiperazine derivative; an organic acid; an ester-based solvent; and an organic sulfoxide.
[2] The transdermal preparation according to [1], in which the phenylpiperazine derivative is at least one selected from aripiprazole or brexpiprazole.
[3] The transdermal preparation according to [1] or [2], in which a content of the organic acid is in a range of 5 parts by mass to 10 parts by mass, a content of the ester-based solvent is in a range of 15 parts by mass to 30 parts by mass, and a content of the organic sulfoxide is in a range of 60 parts by mass to 80 parts by mass with respect to the total content of the organic acid, the ester-based solvent, and the organic sulfoxide.
[4] The transdermal preparation according to any one of [1] to [3], in which a content of the phenylpiperazine derivative is in a range of 5 parts by mass to 11 parts by mass with respect to the total content of the phenylpiperazine derivative, the organic acid, the ester-based solvent, and the organic sulfoxide.

According to an embodiment of the present invention of the present invention, it is possible to provide a transdermal preparation which enables a phenylpiperazine derivative serving as an active component to be transdermally absorbed at a high permeation rate and is capable of maintaining the supply concentration and the supply time of the active component into a living body.

### BRIEF DESCRIPTION OF THE DRAWINGS

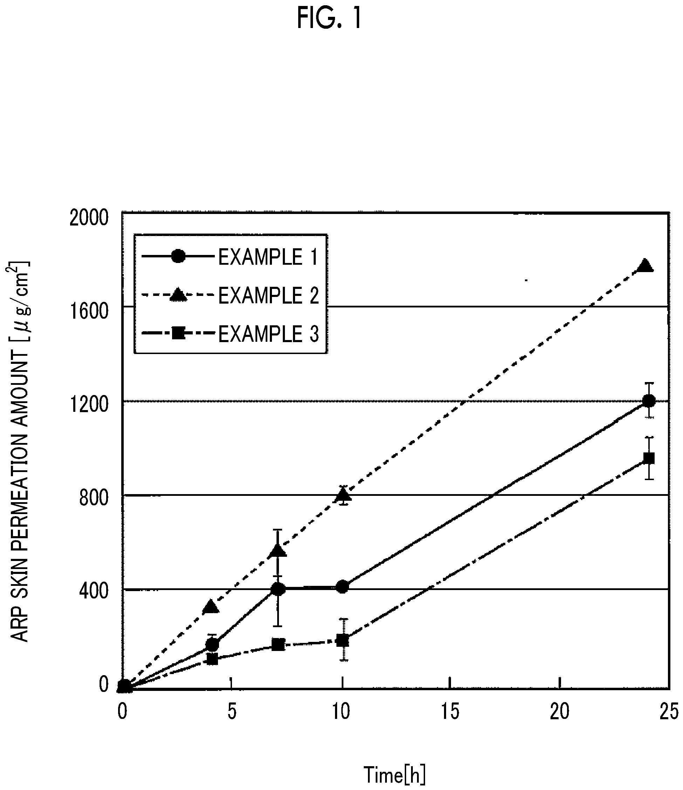

Fig. 1 is a graph showing the results of a test for transdermal absorbability of the transdermal preparations obtained in Examples 1 to 3.

### DESCRIPTION OF THE PREFERRED EMBODIMENTS

Hereinafter, a specific embodiment of the present invention will be described in detail, but the present invention is not limited to the following embodiment and modifications can be appropriately made within the range of the purpose of the present invention.

A transdermal preparation according to the present disclosure (hereinafter, also referred to as the transdermal preparation) is a transdermal preparation containing a phenylpiperazine derivative; an organic acid; an ester-based solvent; and an organic sulfoxide.

The transdermal preparation of the present disclosure is a transdermal preparation which enables a phenylpiperazine derivative serving as an active component to be transdermally absorbed at a high permeation rate and is capable of maintaining the supply concentration and the supply time of the active component into a living body.

Further, "supplying an active component into the living body" in the present specification means that the active component is supplied into the skin and preferably into the blood.

The operational effect of the transdermal preparation of the present disclosure is not clear, but is assumed as follows.

The phenylpiperazine derivative serving as an active component which is contained in the transdermal preparation is useful as a therapeutic agent of schizophrenia, mania or depression in bipolar disorder, or a depression state (hereinafter, also referred to as schizophrenia or the like). The phenylpiperazine derivative is known to have longer half-life in blood compared to those of other drugs in a case where the phenylpiperazine derivative is taken into the body. From the viewpoint of maintaining the drug efficacy, it is desired that the phenylpiperazine derivative maintains the concentration sufficient to ensure the efficacy in the blood.

WO2010/146872A described above describes that the solubility of the phenylpiperazine derivative in an organic acid is high, and it has been examined that a predetermined organic acid and another solvent are used together for dissolving the phenylpiperazine derivative. However, in a case where the penetration rate of another solvent coexisting with the organic acid is higher than that of the organic acid, only the coexisting solvent penetrates into a deep portion under the skin such as the dermis or the epidermis earlier. Accordingly, it is difficult to maintain the solubility of the phenylpiperazine derivative within an appropriate range for an appropriate period of time even in a case where the organic acid remains. Therefore, according to a known technique, it is difficult to continuously supply a required active component concentration into the living body, specifically, into the skin and preferably into the blood for a required time.

The transdermal preparation of the present disclosure contains an ester-based solvent as a solvent of the phenylpiperazine derivative. In a case where the transdermal preparation contains an ester-based solvent, the permeability of three components which are the phenylpiperazine derivative, the organic sulfoxide, and the organic acid into the skin is improved.

The solubility of the phenylpiperazine derivative in a mixed solvent of the organic acid and the organic sulfoxide penetrated into the skin is excellent, the phenylpiperazine derivative is present in a solution in a state of an organic acid salt by being dissolved. Therefore, precipitation of the phenylpiperazine derivative is suppressed, and the penetrability of the active component into the body and preferably into the blood becomes excellent. Here, the penetration rate of the organic sulfoxide contained as a solvent into the dermis or the epidermis is not extremely high as in a known amide-based solvent or the like and is relatively gentle, and the penetration rate is within an appropriate range. Accordingly, it is considered that the high solubility of the phenylpiperazine derivative to be obtained is maintained due to the coexistence of the organic sulfoxide with the organic acid, and the required active component concentration is maintained in the living body and preferably in the blood for a required time at the time of application of the transdermal preparation onto the skin and even after penetration of the transdermal preparation into the skin.

With the above-described composition, since the supply concentration and the supply time of the active component into the living body can be maintained at an appropriate level, the transdermal preparation also has an advantage that the thickness and the area of the transdermal preparation to be attached onto the skin can be made smaller.

Further, the present disclosure is not particularly limited to the above-described assumed mechanism.

The numerical ranges shown using "to" in the present specification indicate ranges including the numerical values described before and after "to" as minimum values and maximum values.

Further, in a case where a composition contains a plurality of substances corresponding to each component, the amount of each component contained in the composition of the present specification indicates the total amount of the plurality of substances unless otherwise specified.

In the numerical ranges described in a stepwise manner in the present specification, an upper limit or a lower limit described in one numerical range may be replaced with an upper limit or a lower limit of another numerical range described in a stepwise manner. Further, in the numerical ranges described in the present specification, an upper limit or a lower limit of the numerical range thereof may be replaced with a value described in the examples.

In the present specification, a combination of preferable forms is a more preferable form.

Hereinafter, each component contained in the transdermal preparation of the present embodiment will be described in detail.

### [Phenylpiperazine derivative]

The transdermal preparation of the present disclosure contains a phenylpiperazine derivative as an active component.

The phenylpiperazine derivative which can be used in the transdermal preparation is not particularly limited as long as the phenylpiperazine derivative is effective for treating schizophrenia or the like.

The molecular weight of the phenylpiperazine derivative is preferably 300 or greater and more preferably 400 or greater.

The upper limit of the molecular weight thereof is not particularly limited, but can be set to 600 or less from the viewpoint of the solubility of the phenylpiperazine derivative in the coexisting solvent.

Examples of the phenylpiperazine derivative suitably used in the transdermal preparation of the present disclosure will be described together with the molecular weight thereof. The numerical value in the parentheses of each component indicates the molecular weight of the corresponding component.

Examples of the phenylpiperazine derivative having a molecular weight of 400 or greater include aripiprazole (molecular weight: 448.38), brexpiprazole (433), etoperidone (414.37), nefazodone (506.46), trazodone (371.8), and vilazodone (441.52).

Among these, at least one selected from aripiprazole (hereinafter, also referred to as ARP) or brexpiprazole is preferable as the phenylpiperazine derivative.

One or two or more kinds of phenylpiperazine derivatives may be contained in the transdermal preparation of the present disclosure.

From the viewpoint of the effects of the present invention, a content of the phenylpiperazine derivative in the transdermal preparation of the present disclosure is preferably 3 parts by mass or greater, more preferably from 5 parts by mass to 11 parts by mass, still more preferably from 6 parts by mass to 11 parts by mass, and particularly preferably from 8 parts by mass to 11 parts by mass with respect to a total content of the transdermal preparation with the coexisting solvent, that is, 100 parts by mass of a total content of the phenylpiperazine derivative, the organic acid, the ester-based solvent, and the organic sulfoxide.

In a case where the content of the phenylpiperazine derivative is in the above-described range and the transdermal preparation of the present disclosure is applied onto the skin, it can be expected that the phenylpiperazine derivative is maintained under the skin and preferably in the blood at a concentration effective for obtaining the drug efficacy for a desired time.

The content of the phenylpiperazine derivative can be measured using high performance liquid chromatography (hereinafter, also referred to as HPLC). As an HPLC measuring device to be used, a device or the like as described below can be used.

### [Organic acid]

The organic acid contained in the transdermal preparation of the present disclosure is not particularly limited. As the organic acid, an organic acid functioning as a solvent of the phenylpiperazine derivative (hereinafter, also referred to as an active component) is preferable. Specifically, it is preferable that the active component can be in the form of a salt of the organic acid in the transdermal preparation due to the presence of the organic acid.

For example, in a case where the phenylpiperazine derivative is present as a salt of the organic acid, the solubility of the active component in the transdermal preparation is further improved. In addition, even after the transdermal preparation is penetrated into the keratin, precipitation of crystals in the body is suppressed so that the active component can be continuously present at an effective concentration for a long period of time.

Examples of the organic acid include a substituted aliphatic carboxylic acid and a substituted aromatic carboxylic acid which contain at least one selected from a hydroxyl group, an alkoxy group, an acyl group, or a ketone group as a substituent.

From the viewpoint of excellent solubility of the active component, among the above-described substituents to be introduced into an aliphatic carboxylic acid or an aromatic carboxylic acid, a hydroxyl group is preferable.

Hereinafter, examples of the aliphatic carboxylic acid and the aromatic carboxylic acid into which the above-described substituents can be introduced will be described.

As the aliphatic carboxylic acid, any of an aliphatic monocarboxylic acid or an aliphatic dicarboxylic acid may be employed.

Examples of the aliphatic monocarboxylic acid include a short-chain aliphatic monocarboxylic acid having 2 to 7 carbon atoms, a medium-chain aliphatic monocarboxylic acid having 8 to 11 carbon atoms, and a long-chain aliphatic monocarboxylic acid having 12 or more carbon atoms.

Examples of the short-chain aliphatic monocarboxylic acid having 2 to 7 carbon atoms include acetic acid, butyric acid, hexanoic acid, and cyclohexanecarboxylic acid. Examples of the medium-chain aliphatic monocarboxylic acid having 8 to 11 carbon atoms include octanoic acid and decanoic acid. Examples of the long-chain aliphatic monocarboxylic acid having 12 or more carbon atoms include myristic acid, stearic acid, isostearic acid, and oleic acid.

Examples of the aliphatic dicarboxylic acid include sebacic acid, adipic acid, malic acid, maleic acid, and fumaric acid.

Examples of the aromatic carboxylic acid include benzoic acid and cinnamic acid.

Examples of the aromatic carboxylic acid having a substituent include p-hydroxybenzoic acid, salicylic acid, acetylsalicylic acid.

Among the examples, an aliphatic monocarboxylic acid having the above-described substituent is preferable, and a short-chain aliphatic monocarboxylic acid having the above-described substituent and 2 to 7 carbon atoms is more preferable. More specific examples thereof include glycolic acid, lactic acid, methoxyacetic acid, mandelic acid, levulinic acid, and 3-hydroxybutyric acid. From the viewpoints of the solubility of the active component and the biocompatibility, a short-chain aliphatic monocarboxylic acid having a hydroxyl group as a substituent and 2 to 7 carbon atoms is more preferable. Among the above-described examples, lactic acid or glycolic acid is still more preferable.

In a case where the solvent of the active component which can be contained in the transdermal preparation of the present disclosure contains an organic acid and the ester-based solvent and the organic sulfoxide described below, the solubility of the active component, the stability, and the absorption rate in the transdermal absorption are respectively in the preferable range as described above.

A content of the organic acid is preferably from 5 parts by mass to 10 parts by mass, more preferably from 7 parts by mass to 10 parts by mass, and still more preferably from 8 parts by mass to 10 parts by mass with respect to 100 parts by mass of a total content of the organic acid, the ester-based solvent, and the organic sulfoxide.

The transdermal preparation of the present disclosure may contain one or two or more kinds of organic acids.

The content of the organic acid in the transdermal preparation can be measured according to a known method.

For example, the content of the organic acid can be measured using ion chromatography. The measurement performed using ion chromatography can be carried out with, for example, ICS-2000 (manufactured by Nippon Dionex K.K.). The prepared transdermal preparation is diluted using 1 mM KOH as an eluent, and the measurement using ion chromatography can be performed.

### [Ester-based solvent]

In a case where the transdermal preparation of the present disclosure contains an ester-based solvent, the penetrability of the active component in the transdermal preparation, the organic acid serving as a coexisting solvent, and the organic sulfoxide into the keratin is further improved, and solvent distribution in the dermis or the epidermis under the keratin can be further improved.

Examples of the ester-based solvent include alkyl ester of an aliphatic dicarboxylic acid. Specific examples of the ester-based solvent include triacetin, diethyl sebacate, diisopropyl adipate, diisopropyl sebacate, isopropyl palmitate, and isopropyl myristate. Among the examples of the ester-based solvent, from the viewpoint of excellent penetrability of the active component and the like into the keratin, diethyl sebacate or diisopropyl adipate is preferable, and diethyl sebacate is more preferable.

The transdermal preparation of the present disclosure may contain only one or two or more kinds of ester-based solvents.

From the viewpoint of further improving the penetrability into the keratin and the solvent distribution, a content of the ester-based solvent in the transdermal preparation of the present disclosure is preferably from 15 parts by mass to 30 parts by mass, more preferably from 20 parts by mass to 30 parts by mass, and still more preferably from 25 parts by mass to 30 parts by mass with respect to 100 parts by mass of a total content of the organic acid, the ester-based solvent, and the organic sulfoxide.

Further, the content of the ester-based solvent in the transdermal preparation can be measured according to a known method.

For example, the content of the ester-based solvent in the transdermal preparation can be measured using a gas chromatograph mass spectrometer (GC-MS). For example, GCMS-QP2010 (manufactured by Shimadzu Corporation) can be used as GC-MS. The measurement can be performed by diluting the prepared transdermal preparation with a diluent such as ethanol or the like.

### [Organic sulfoxide]

The transdermal preparation of the present disclosure contains an organic sulfoxide.

In a case where the transdermal preparation contains an organic sulfoxide, it is speculated that the solubility of the active component in the transdermal preparation is further improved so that the active component whose penetrability into the dermis and the epidermis is maintained at an appropriate speed is supplied at a required concentration for a required time, which means that the active component in the transdermal preparation is continuously supplied into the dermis and the epidermis and, further, into the skin and preferably to the blood.

Examples of the organic sulfoxide in the present disclosure include a compound represented by Formula (I), sulfolane, and dimethylsulfone.

The compound represented by formula (I) will be described.

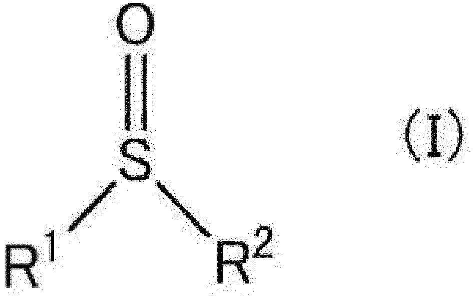

In Formula (I), R¹ and R² each independently represent a monovalent organic group. R¹ and R² may be bonded to each other to form a ring structure.

Examples of the monovalent organic group as R¹ and R² include an alkyl group and an aryl group. From the viewpoints of further improving the solubility of the active component and controlling the penetration rate to be in an appropriate range, an alkyl group having 1 to 5 carbon atoms is preferable as the monovalent organic group in Formula (I).

Among the examples of the compound represented by formula (I), dimethyl sulfoxide (hereinafter, also referred to as DMSO) or the like in which both of R¹ and R² represent a methyl group is more preferable.

As the organic sulfoxide which can be used in the transdermal preparation of the present disclosure, at least one selected from dimethyl sulfoxide, sulfolane, or dimethyl sulfone is preferable and dimethyl sulfoxide is more preferable.

The transdermal preparation of the present disclosure may contain only one or two or more kinds of organic sulfoxides.

From the viewpoints of respectively maintaining the solubility of the active component and the penetrability of the active component to be in an appropriate range, a content of the organic sulfoxide in the transdermal preparation of the present disclosure is preferably from 60 parts by mass to 80 parts by mass, more preferably from 60 parts by mass to 70 parts by mass, and still more preferably from 60 parts by mass to 65 parts by mass with respect to 100 parts by mass of a total content of the organic acid, the ester-based solvent, and the organic sulfoxide.

The content of the organic sulfoxide is not particularly limited and can be measured according to a known method.

The content of the organic sulfoxide can be measured using GC-MS. For example, GCMS-QP2010 (manufactured by Shimadzu Corporation) can be used as GC-MS. The content of the organic sulfoxide can be measured by diluting the prepared transdermal preparation with a diluent such as ethanol or the like using GC-MS.

It is preferable that the transdermal preparation of the present disclosure contains from 5 parts by mass to 10 parts by mass of the organic acid, from 15 parts by mass to 30 parts by mass of the ester-based solvent, and from 60 parts by mass to 80 parts by mass of the organic sulfoxide with respect to 100 parts by mass of a total content of the organic acid, the ester-based solvent, and the organic sulfoxide.

As the balance of the content ratio of the solvents contained in the transdermal preparation, the content ratio of the solvents (organic acid:ester-based solvent:organic sulfoxide) is preferably 5 to 10:10 to 35:60 to 80 and more preferably 5 to 10:15 to 35:60 to 80 in terms of mass ratio. Further, more specifically, the content ratio of the solvents (organic acid:ester-based solvent:organic sulfoxide) can be set to, for example, 5:35:60, 10:30:60, 5:15:60, or 10:10:80.

[Other components which can be contained in transdermal preparation]

The transdermal preparation of the present disclosure may contain known additives in accordance of the dosage form of the transdermal preparation depending on the intended purpose thereof within the range not damaging the effects of the present invention, in addition to the above-described active components and solvents.

Examples of the additives which can be used in the transdermal preparation of the present disclosure include a transdermal absorption promotor, a wetting agent, a skin softener, a skin protective agent, a base, a surfactant, a thickener, a gelling agent, a softening agent, a filler, organic particles, inorganic particles, a buffer, a pH adjuster, a colorant, and a flavoring agent. Further, for the purpose of improving the stability of the transdermal preparation, the transdermal preparation may contain a known stabilizer and a known antioxidant.

Examples of the softening agent include a petroleum-based softening agent such as process oil or low-molecular weight polybutene, a fatty acid-based softening agent such as coconut oil or castor oil, and purified lanolin.

Examples of the filler include zinc oxide, titanium oxide, calcium carbonate, and silicic acids.

Examples of the components which can be used for adjusting the viscosity of the preparation such as a gelling agent and a thickener include a cellulose derivative such as methyl cellulose, ethyl cellulose, or hydroxypropyl methyl cellulose, a carboxy vinyl polymer, and polyvinyl alcohol.

### [Preparation of transdermal preparation]

The transdermal preparation of the present disclosure can be produced according to a conventional method. An example of a method of producing the transdermal preparation is as follows.

The organic sulfoxide, the ester-based solvent, and the organic acid are weighed in a container and sufficiently mixed to prepare a mixed solvent.

Thereafter, a required amount of the phenylpiperazine derivative is weighed, added to the mixed solvent obtained according to the above-described method, further stirred, and sufficiently dissolved therein, thereby obtaining a transdermal preparation. The transdermal preparation can be prepared by stirring the solution at room temperature (25°C).

Further, in a case where a preparation with a composition in which the dissolution rate of the phenylpiperazine derivative or the like is low is prepared, for example, after the phenylpiperazine derivative is added to the mixed solvent, the solution may be heated to 40°C to 70°C and stirred, the dissolution may be promoted by performing an ultrasonic treatment, or both of the heating and the ultrasonic treatment may be performed. The transdermal preparation produced by performing the dissolution promoting treatment is allowed to stand at room temperature for one day, and it is preferable that insoluble matter, a precipitate, or the like is not found after the standing of the transdermal preparation for one day and the dissolution of the active components is visually confirmed.

In a case where the transdermal preparation contains other components, each component can be contained in the preparation using a conventional method at a stage suitable for each component.

The viscosity of the transdermal preparation can be adjusted by allowing the transdermal preparation in a liquid state to contain a gelling agent, a thickener, a softening agent, a filler, and the like.

The viscosity of the transdermal preparation in a liquid state can be adjusted according to a conventional method. As a method of adjusting the viscosity, for example, a method of adding at least one selected from the above-described components which can be used for adjusting the viscosity to the transdermal preparation in a liquid state obtained in the above-described manner according to the target viscosity, sufficiently mixing the transdermal preparation while heating the same, and naturally cooling the transdermal preparation is exemplified.

### [Dosage form of transdermal preparation]

The dosage form of the transdermal preparation of the present disclosure is not particularly limited as long as the above-described active components can be held on the skin for a desired time.

Examples of the dosage form of the transdermal preparation of the present disclosure include a liquid drug, a gelling agent, an ointment, a cream, and a reservoir type patch. The shape of the reservoir type patch is not particularly limited.

The liquid drug, the gelling agent, the ointment, the cream, or the like can be used by adjusting the viscosity thereof according to the intended purpose thereof and being applied onto the skin as it is. Further, the gelling agent, the ointment, the cream, or the like can be used by being applied to a support such as non-woven fabric or a bandage and bringing the application surface into contact with the skin.

As another form of using a skin preparation such as a liquid drug or a gelling agent, a reservoir type patch is exemplified.

In other words, in a case where the transdermal preparation has a dosage form of a liquid drug, a gelling agent, an ointment, or a cream with a relatively low viscosity and has fluidity, a base material such as non-woven fabric, woven fabric, or a sponge having communication bubbles is impregnated with the preparation in the dosage form having fluidity to form a drug storage layer, the drug storage layer is disposed on a support, and a surface of the drug storage layer on a side opposite to a side where the support is provided is coated with a film which is capable of controlling the release of the transdermal preparation, thereby obtaining a reservoir type patch.

In a case where the transdermal preparation is a preparation which has a higher viscosity and lower fluidity compared to a liquid drug, similar to a gelling agent or an ointment, the transdermal preparation is applied directly onto a support to form a transdermal preparation layer, and a surface of the formed transdermal preparation layer on a side opposite to a side where the support is provided is coated with a film which is capable of controlling the release of the transdermal preparation, thereby obtaining a reservoir type patch.

In the reservoir type patch, the transdermal preparation is gradually released from fine opening portions of a membrane positioned on the surface of the drug storage layer or the transdermal preparation layer, that is, the surface in contact with the skin to the skin.

Examples of the structure of the reservoir type patch described above are described in paragraphs [0007] to [0022] and Fig. 1 of JP2003-063954A and can be referred to in the present disclosure.

### (Transdermal absorbability of phenylpiperazine derivative in transdermal preparation)

Since the transdermal preparation of the present disclosure contains a phenylpiperazine derivative serving as an active component which has excellent transdermal absorbability and the phenylpiperazine derivative can be continuously administered, excellent drug efficacy can be maintained for a desired time.

As a method of evaluating the transdermal absorbability, a known skin permeability evaluation method is exemplified. The skin permeability can be evaluated according to an in vitro skin permeation experimental method described below.

Further, the skin permeability evaluated according to a known skin permeability experimental method has the same definition as that for the transdermal absorbability of the transdermal preparation in the present specification.

As the in vitro skin permeation experimental method, a method of using a diffusion cell is exemplified. Examples of the diffusion cell include vertical cells such as Franz type diffusion cells and horizontal cells. The diffusion cell is formed of two cell parts and a membrane that measures the permeability is interposed between two cell parts. Examples of the membrane include the human skin, the animal skin, a three-dimensional cultured skin model, and an artificial membrane.

In the present specification, as an example of the skin permeability evaluation method, the evaluation is made by performing a skin permeability test using the excised skin of rats described below.

### (Example of evaluation method)

For example, a test for evaluating the transdermal absorbability can be performed under the following conditions using Franz diffusion cells (permeation area: 1 cm², volume of receptor solution: 8 mL) at a test temperature of 32°C.

The abdominal excised skin of 8-week-old specific pathogen free (SPF) rats is used as a membrane.

As the receptor solution, a solution containing polyethylene glycol 400 and PBS (pH of 7.4) at a mixing ratio of 6:4 is used.

The concentration of the active components permeating through the skin is measured using high performance liquid chromatography (HPLC: prominence, manufactured by Shimadzu Corporation).

The commercially available abdominal excised skin of rats described above is interposed between vertical diffusion cells (effective diffusion area: 1 cm²), the transdermal preparation serving as a test object is applied to the keratin side, and the above-described receptor solution is applied to the dermis side.

After the experiment is started, 300 µL of the receptor solution is sampled at the 4th hour, the 7th hour, the 10th hour, (the 22nd hour), and the 24th hour, the concentration of the phenylpiperazine derivative eluted after permeation into the skin is measured using HPLC described above, and the accumulated permeation amount of the drug at each hour is measured.

Further, examples of an in vivo skin permeation experimental method include a skin pharmacokinetic test, a biological test, a residual amount test, a kinetic test, a clinical test, an animal test, and an exposed amount test.

### <Use of transdermal preparation>

The transdermal preparation of the present disclosure is used by being administered to the skin of a subject for application.

Since a desired amount of the active component is usually allowed to be penetrated into the skin over time, as described above, the transdermal preparation impregnated into a base material is applied to the subject by being fixed onto the skin or applied as a reservoir type patch. Examples of the base material which can be used as a support in the reservoir type patch include a resin film having air permeability, non-woven fabric, cloth, and a film which does not have air permeability.

In a case where the transdermal preparation of the present disclosure is disposed on one surface of the support to obtain a reservoir type patch, the side where the transdermal preparation is disposed becomes the surface to adhere to the surface.

A single dose of the phenylpiperazine derivative contained in the transdermal preparation and the number of times of administration per day can be appropriately selected according to the intended purpose thereof. In other words, the amount and the number of times that enable a required amount of phenylpiperazine derivative to be absorbed by the skin of the subject for application are selected. Typically, a single dose of the transdermal preparation is approximately 3 mg to 6 mg and the number of times of administration per day is approximately once or twice. Further, the dose and the number of times of administration are not limited to the above-described range.

Since the transdermal preparation of the present disclosure is transdermally absorbed at a high permeation rate and rapidly penetrated into the keratin, the penetration rate in the dermis and the epidermis is in an appropriate range, the supply concentration and the supply time of the active components into the living body (under the skin and preferably in the blood) can be maintained at an appropriate level. Therefore, the phenylpiperazine derivative serving as an active component can be continuously penetrated into the skin for a certain time so that the phenylpiperazine derivative is supplied into the body from a region where a patch is fixed.

### <Treatment method>

Other embodiments of the present disclosure include a method of treating schizophrenia, including transdermally administering the above-described transdermal preparation that contains a phenylpiperazine derivative serving as an active component to a subject for application serving as a target for treating the schizophrenia or the like described above.

### Examples

Hereinafter, the transdermal preparation of the present disclosure will be described in detail with reference to the following examples. However, the transdermal preparation of the present disclosure is not particularly limited to the following examples, and various embodiments can be employed within the range not departing from the scope of the present invention.

### [Example 1]

Based on the composition listed in Table 1, dimethyl sulfoxide (DMSO) which is an organic sulfoxide, ethyl sebacate (DES) which is an ester-based solvent, and lactic acid (LA) which is an organic acid were weighed in a container and sufficiently stirred to prepare a mixed solvent. In addition, the content ratio of the solvents listed in Tables 1 and 2 are on a mass basis.

Thereafter, aripiprazole (ARP) which is a phenylpiperazine derivative was weighed, was added to the mixed solvent obtained according to the above-described method, further stirred, and then sufficiently dissolved therein, thereby obtaining a transdermal preparation of Example 1. The preparation was prepared by stirring the mixed solvent at room temperature (25°C).

It was confirmed that insoluble matter, a precipitate, or the like was not found in the obtained transdermal preparation through visual observation and the active components were uniformly dissolved.

**[Table 1]**

| | | Molecular weight (MW (g/mol)) | Content (g) | Content (% by mass) | Content ratio (DMSO:DES:LA) |
|---|---|---|---|---|---|
| Example 1 | Aripiprazole (ARP) | 448 | 1.1 | 11 | 65:30:5 |
| | Dimethyl sulfoxide (DMSO) | 78 | 5.785 | 57.85 | |
| | Diethyl sebacate (DES) | 258 | 2.67 | 26.7 | |
| | Lactic acid (LA) | 90 | 0.445 | 4.45 | |
| | Total amount | | 10 | 100 | |

### [Examples 2 to 4]

Based on the compositions listed in Table 2, transdermal preparations of Examples 2 to 4 were prepared in the same manner as in Example 1. It was confirmed that insoluble matter, a precipitate, or the like was not found in all of the transdermal preparations through visual observation and the active components were uniformly dissolved.

Further, all of the molecular weights of ARP, DMSO, DES, and LA listed in Table 2 are the same as the molecular weights listed in Table 1.

**[Table 2]**

| | | Content (g) | Content (% by mass) | Content ratio (DMSO:DES:LA) |
|---|---|---|---|---|
| Example 2 | Aripiprazole (ARP) | 1.1 | 11 | 60:30:10 |
| | Dimethyl sulfoxide (DMSO) | 5.34 | 53.4 | |
| | Diethyl sebacate (DES) | 2.67 | 26.7 | |
| | Lactic acid (LA) | 0.89 | 8.9 | |
| | Total amount | 10 | 100 | |
| Example 3 | Aripiprazole (ARP) | 1.1 | 11 | 80:15:5 |
| | Dimethyl sulfoxide (DMSO) | 7.12 | 71.2 | |
| | Diethyl sebacate (DES) | 1.335 | 13.35 | |
| | Lactic acid (LA) | 0.445 | 4.45 | |
| | Total amount | 10 | 100 | |
| Example 4 | Aripiprazole (ARP) | 0.5 | 5 | 60:30:10 |
| | Dimethyl sulfoxide (DMSO) | 5.7 | 57 | |
| | Diethyl sebacate (DES) | 2.85 | 28.5 | |
| | Lactic acid (LA) | 0.95 | 9.5 | |
| | Total amount | 10 | 100 | |

The details of each component described in Tables 1, 2, and 4 are as follows.
- Aripiprazole (Tokyo Chemical Industry Co., Ltd.)
- Dimethyl sulfoxide (Wako Pure Chemical Industries, Ltd., used for molecular biology)
- Diethyl sebacate (Nikko Chemicals Co., Ltd., NIKKOL (registered trademark) DES-SP)
- Lactic acid (Merck KGaA)

### [Evaluation]

The transdermal absorbability of each transdermal preparation of Examples 1 to 4, obtained in the above-described manner, was evaluated according to the following method.

A test for evaluating the transdermal absorbability was performed under the following conditions using Franz diffusion cells (permeation area: 1 cm², volume of receptor solution: 8 mL) at a test temperature of 32°C.

The abdominal excised skin of 8-week-old specific pathogen free (SPF) rats was used as a membrane.

As the receptor solution, a solution containing ethylene glycol 400 and PBS (pH of 7.4) at a mixing ratio of 6:4 was used.

The concentration of the active components permeating through the skin was measured using high performance liquid chromatography (HPLC: prominence, manufactured by Shimadzu Corporation).

The commercially available abdominal excised skin of rats described above was interposed between vertical diffusion cells (effective diffusion area: 1 cm²), 113 mg/cm² of each transdermal preparation serving as a test object was applied to the keratin side, and the above-described receptor solution is applied to the dermis side.

After the experiment was started, 300 µL of the receptor solution was sampled at the 4th hour, the 7th hour, the 10th hour, (the 22nd hour), and the 24th hour, the concentration of aripiprazole eluted after permeation into the skin was measured using HPLC described above, and the accumulated permeation amount of ARP at each hour was measured. The results are listed in Table 3. In Table 3, "-" indicates that the measurement was not performed. Further, the results obtained by measuring the skin permeation amounts of Examples 1 to 3, each of which has the same level of content of ARP from among the components, are shown in the graph of Fig. 1.

**[Table 3]**

| | Amount of transdermal preparation to be administered (mg/cm²) | ARP concentration in transdermal preparation (% by mass) | Content ratio (DMSO:DES: LA) | Skin permeation amount (accumulated amount: µg/cm²) | | | | |
|---|---|---|---|---|---|---|---|---|
| | | | | After 4 hours | After 7 hours | After 10 hours | After 22 hours | After 24 hours |
| Example 1 | 113 | 11 | 60:35:5 | 175.25 | 408.89 | 411.27 | - | 1212.13 |
| Example 2 | 113 | 11 | 60:30:10 | 125.29 | 294.09 | 411.18 | 938.53 | 1017.23 |
| Example 3 | 113 | 11 | 80:15:5 | 115.88 | 174.96 | 195.57 | - | 965.05 |
| Example 4 | 113 | 5 | 60:30:10 | 71.59 | 223.01 | 383.42 | 1206.86 | 1269.32 |

As shown in the results listed in Table 3, in all of the transdermal preparations of Examples 1 to 4, the skin permeability (transdermal absorbability) of ARP serving as an active component was excellent and the skin permeation accumulated amount of ARP was increased with time up to 24 hours after applications of the transdermal preparation. Further, it can be confirmed, even in Fig. 1, that each graph has a certain inclination and the accumulated amount increases. As shown in these results, it was found that the supply of a predetermined amount of ARP into the skin was maintained for at least 24 hours by permeation of ARP into the skin in the case of the transdermal preparation of each example.

### [Example 5 and Comparative Examples 1 and 2]

In the transdermal preparation of the present disclosure, for the purpose of confirming the effects of the solvent, transdermal preparations of Example 5, Comparative Example 1, and Comparative Example 2, in which the content of ARP was set to 3.75% by mass and different solvents were used, were respectively prepared in the same manner as in Example 1 based on the formulations described in Table 4.

It was confirmed that insoluble matter, a precipitate, or the like was not found in the obtained transdermal preparation of Example 5 through visual observation and the active components were uniformly dissolved.

It was confirmed that insoluble matter, a precipitate, or the like was not found in both of the transdermal preparations of Comparative Examples 1 and 2 through visual observation and the active components were uniformly dissolved.

**[Table 4]**

| | | Content (g) | Content (% by mass) | Content ratio (DMSO:DES:LA) |
|---|---|---|---|---|
| Example 5 | Aripiprazole (ARP) | 0.375 | 3.75 | 10:10:80 |
| | Dimethyl sulfoxide (DMSO) | 0.963 | 9.625 | |
| | Diethyl sebacate (DES) | 0.963 | 9.625 | |
| | Lactic acid (LA) | 7.700 | 77 | |
| | Total amount | 10 | 100 | |
| Comparative Example 1 | Aripiprazole (ARP) | 0.375 | 3.75 | 50:0:50 |
| | Dimethyl sulfoxide (DMSO) | 4.8125 | 48.125 | |
| | Diethyl sebacate (DES) | 0 | 0 | |
| | Lactic acid (LA) | 4.8125 | 48.125 | |
| | Total amount | 10 | 100 | |
| Comparative Example 2 | Aripiprazole (ARP) | 0.375 | 3.75 | 0:0:100 |
| | Dimethyl sulfoxide (DMSO) | 0 | 0 | |
| | Diethyl sebacate (DES) | 0 | 0 | |
| | Lactic acid (LA) | 9.625 | 96.25 | |
| | Total amount | 10 | 100 | |

The transdermal absorbability of each transdermal preparation of Example 5, Comparative Example 1, and Comparative Example 2, obtained in the above-described manner, was evaluated in the same manner as in Example 1, and the skin permeation accumulated amount of ARP after 24 hours was measured.

The results are listed in Table 5.

**[Table 5]**

| | Amount of transdermal preparation to be administered (mg/cm²) | ARP concentration in transdermal preparation (% by mass) | Content ratio (DMSO:DES: LA) | Skin permeation amount (accumulated amount: µg/cm²) |
|---|---|---|---|---|
| | | | | After 24 hours |
| Example 5 | 113 | 3.75 | 10:10:80 | 890.55 |
| Comparative Example 1 | 113 | 3.75 | 50:0:50 | 4.44 |
| Comparative Example 2 | 113 | 3.75 | 0:0:100 | 36.28 |

As listed in Table 5, in Example 5, the skin permeation accumulated amount of ARP after 24 hours was sufficiently large even in a case of a skin preparation having a low content of ARP. Further, it was understood that the skin permeation accumulated amount of Comparative Examples 1 and 2, in which the transdermal preparation did not contain any of DMSO, DES, and LA, was significantly smaller than that of Example 5 even in a case where lactic acid (LA) known to have excellent solubility of ARP was contained in the solvent.

The disclosure of JP No. 2016-096178 filed on May 12, 2016 and the disclosure of JP No. 2016-245702 filed on December 19, 2016 are incorporated in the present specification by reference.

All documents, patent applications, and technical standards described in the present specification are incorporated herein by reference to the same extent as a case of being specifically and individually noted that individual documents, patent applications, and technical standards are incorporated by reference.

## Claims

1. A transdermal preparation comprising:
a phenylpiperazine derivative;
an organic acid;
an ester-based solvent; and
an organic sulfoxide.

2. The transdermal preparation according to claim 1, wherein the phenylpiperazine derivative is at least one selected from aripiprazole or brexpiprazole.

3. The transdermal preparation according to claim 1 or 2, wherein, with respect to 100 parts by mass of a total content of the organic acid, the ester-based solvent, and the organic sulfoxide, a content of the organic acid is from 5 parts by mass to 10 parts by mass, a content of the ester-based solvent is from 15 parts by mass to 30 parts by mass, and a content of the organic sulfoxide is from 60 parts by mass to 80 parts by mass.

4. The transdermal preparation according to any one of claims 1 to 3, wherein a content of the phenylpiperazine derivative is from 5 parts by mass to 11 parts by mass with respect to 100 parts by mass of a total content of the phenylpiperazine derivative, the organic acid, the ester-based solvent, and the organic sulfoxide.
